# EUROPEAN PATENT APPLICATION

(11) **EP 4 755 869 A1**
(43) Date of publication of application: **10.06.2026**
(21) Application number: 24217827.5
(22) Date of filing: 05.12.2024
(51) Int. Cl.: C07C 47/228, C07C 47/235, A61K 8/00, A61Q 13/00, A61Q 19/00, C11D 3/50

(54) **ORGANIC COMPOUND**

(71) Applicant: Givaudan SA, 1214 Vernier (CH)
(72) Inventor: The designation of the inventor has not yet been filed
(74) Representative: Global Patents

(57) **Abstract**

The invention relates to a compound of formula (I) 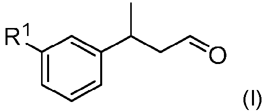 wherein R¹ is selected from n-propyl, n-butyl, n-pentyl, n-hexyl and n-heptyl, which is a useful fragrance ingredient.

## Description

### TECHNICAL FIELD

The present invention relates to novel biodegradable compounds possessing watery-marine, floral-aldehydic olfactory properties. The invention furthermore refers to methods for their production, and to flavour and fragrance compositions and perfumed articles comprising those compounds.

### BACKGROUND

The dihydrocinnamic aldehyde family is of great economic importance in perfumery as outlined by S. Jordi at al (Helv. Chim. Acta, 2018,101, e1800048). There is a transition between muguet (lily of the valley) odor into the watery, marine odor family, and minute structural changes trigger great differences in performance and hedonics. There is a growing regulatory risk associated to CMR concerns in the muguet odorant family. At the same time, many marine compounds (e.g. calone, azurone etc) are not biodegradable. There remains a need to provide further safe floral, marine notes with improved odour profiles to address shrinking options for perfumers to create enticing and facet-rich floralcy in modern perfumery.

Typical perfumery ingredients with marine odour notes are derivatives of 2*H-*benzo[b][1,4]dioxepin-3(4*H*)-one, like Azurone or Calone. In WO2014/205782 a group of *meta-*substituted 3-phenylpropanal derivatives was described. Substituents on the benzoxepinone skeleton can be branched or non-branched as pointed out by J.-M. Gaudin et al (Helv. Chim. Acta 2007, 90, 1245), while in the dihydrocinnamic aldehyde family, only branched alkyl chains provided the olfactory performance necessary to allow industrialization in this competitive field of muguet odorants (Lilial, Bourgeonal, Cyclemax, Silvial etc). It was believed that a substituent in the meta position has to have some complexity to provide the desired hedonics.

### SUMMARY

In accordance with a first aspect of the present invention there is provided a compound of formula (I) 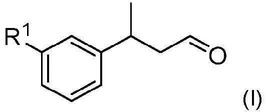 wherein R¹ is selected from *n*-propyl, *n*-butyl, *n*-pentyl, *n*-hexyl and *n*-heptyl.

In accordance with a second aspect of the present invention there is provided a use of the compound of formula (I) as fragrance.

In accordance with a third aspect of the present invention there is provided a fragrance composition comprising at least one compound of formula (I).

In accordance with a forth aspect of the present invention there is provided a consumer product comprising at least one compound of formula (I) and a consumer product base.

In accordance with a fifth aspect of the present invention there is provided a method for improving, enhancing or modifying a consumer product base by means of the addition thereto a compound of formula (I).

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### DETAILED DESCRIPTION

The present invention is based on the surprising finding that small series of 3-(3-*n*-alkyl phenyl)butanals possess very typical and characteristic watery-marine notes.

So in a first aspect of the present invention, there is provided a compound of formula (I) 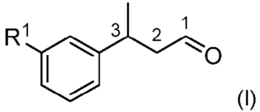 wherein R¹ is selected from *n*-propyl, *n*-butyl, *n*-pentyl, *n*-hexyl and *n*-heptyl.

The compound of the present invention possesses a stereocenter at C3 and in consequence exist as pair of enantiomers, which are both encompassed by the present invention. The chiral compound of formula (I) can be enantiomerically pure, enriched or racemic.

The compound of formula (I) surprisingly possesses not only very typical and characteristic floral, watery-marine notes of remarkable performance but, in addition, provide impressions of outstanding freshness, associated with cleanliness. It appears that a substituent in the *meta-*position is responsible for this odour direction, which is in contrast to other phenyl aldehydes. The length of R¹ is also relevant for the odour and for the substantivity of the compound. If R¹ is shorter than 3C, the odour direction is changed and the substantivity is lost (comparative example 1 and example 9). On the other hand, longer R¹ substituents do not provide the desired marine notes with sufficient performance.

It was surprisingly found that the compounds of formula (I) with linear alkyl residues in *meta-*position have lower the odour threshold values in comparison to compounds bearing branched, unsaturated or cyclic units with the same number of carbon atoms. Some derivatives with branched, unsaturated or cyclic residues are disclosed in WO2014/205782. The comparison of the odour threshold values is given in the examples below.

The presence of the methyl group at C3 position (α-position to benzene ring) of the compound of formula (I) is essential to provide the desired marine notes of the compound. In the absence of said group, the compounds have a metallic aldehydic odour. This effect is demonstrated by comparative examples 2 to 4. In addition, said methyl group at C3 position (α-position to benzene ring) of the compound of formula (I) prevents the formation of the corresponding benzoic acid derivative *in vitro.* Since benzoic acid derivatives cause reproductive toxicity in male rats, these toxic effects are prevented by the methyl substituent at C3 position of the compound of formula (I). In consequence, the compound of formula (I) do not raise any reprotoxic concerns, like the structurally related muguet compound Lilial^{™} (3-(4-tert-butylphenyl)-2-methylpropanal; CAS 80-54-6).

The compound of formula (I) is biodegradable, as demonstrated in the examples below.

It was further shown that the compounds of the present invention do activate a novel olfactory receptor discovered by the applicant. In contrast, similar compounds with branched residues do not activate this receptor. This test and the novel olfactory receptors are more fully described in the examples below, For example, the compound of formula (I) is selected from the group consisting of 3-(3-propylphenyl)butanal, 3-(3-butylphenyl)butanal, 3-(3-pentylphenyl)butanal, 3-(3-hexylphenyl)butanal and 3-(3-heptylphenyl)butanal. In a particular example, the compound of formula (I) is 3-(3-pentylphenyl)butanal.

The compounds of formula (I) may be used alone, as mixtures thereof, or in fragrance compositions in combination with a base material. As used herein, the 'base material' includes all known odorant molecules selected from the extensive range of natural products, and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odorants in fragrance compositions, for example, carrier materials, and other auxiliary agents commonly used in the art.

As used herein, 'fragrance composition' means any composition comprising at least one compound of formula (I) and a base material, e.g. a diluent conventionally used in conjunction with odorants, such as dipropylene glycol (DPG), isopropyl myristate (IPM), triethyl citrate (TEC) and alcohol (e.g. ethanol).

As used herein, "carrier material" means a material which is practically neutral from an odorant point of view, i.e. a material that does not significantly alter the organoleptic properties of odorants.

As used herein, the term "auxiliary agent" refers to ingredients that might be employed in a fragrance composition for reasons not specifically related to the olfactory performance of said composition. For example, an auxiliary agent may be an ingredient that acts as an aid to processing a fragrance ingredient or ingredients, or a composition containing said ingredient(s), or it may improve handling or storage of a fragrance ingredient or composition containing same. It might also be an ingredient that provides additional benefits such as imparting color or texture. It might also be an ingredient that imparts light resistance or chemical stability to one or more ingredients contained in a fragrance composition. A detailed description of the nature and type of adjuvants commonly used in fragrance compositions containing same cannot be exhaustive, but it has to be mentioned that said ingredients are well known to a person skilled in the art.

The following list comprises examples of other odorant molecules, which may be combined with the compounds of the present invention:
- Essential oils and extracts, e.g. castoreum, costus root oil, oak moss absolute, geranium oil, tree moss absolute, basil oil, fruit oils, such as bergamot oil and mandarine oil, myrtle oil, palmarose oil, patchouli oil, petitgrain oil, jasmine oil, rose oil, sandalwood oil, wormwood oil, lavender oil and/ or ylang-ylang oil;
- Alcohols, e.g. cinnamic alcohol ((*E*)-3-phenylprop-2-en-1-ol); cis-3-hexenol ((*Z*)-hex-3-en-1-ol); Citronellol (3,7-dimethyloct-6-en-1-ol); dihydro myrcenol (2,6-dimethyloct-7-en-2-ol); Ebanol^{™} ((*E*)-3-methyl-5-(2,2,3-trimethylcyclopent-3-en-1-yl)pent-4-en-2-ol); eugenol (4-allyl-2-methoxyphenol); ethyl linalool ((*E*)-3,7-dimethylnona-1,6-dien-3-ol); farnesol ((2*E*,6*Z*)-3,7,11-trimethyldodeca-2,6,10-trien-1-ol); geraniol ((*E*)-3,7-dimethylocta-2,6-dien-1-ol); Super Muguet^{™} ((*E*)-6-ethyl-3-methyloct-6-en-1-ol); linalool (3,7-dimethylocta-1,6-dien-3-ol); menthol (2-isopropyl-5-methylcyclohexanol); Nerol (3,7-dimethyl-2,6-octadien-1-ol); phenyl ethyl alcohol (2-phenylethanol); Rhodinol^{™} (3,7-dimethyloct-6-en-1-ol); Sandalore^{™} (3-methyl-5-(2,2,3-trimethylcyclopent-3-en-1-yl)pentan-2-ol); terpineol (2-(4-methylcyclohex-3-en-1-yl)propan-2-ol); or Timberol^{™} (1-(2,2,6-trimethylcyclohexyl)hexan-3-ol); 2,4,7-trimethylocta-2,6-dien-1-ol, and/or [1-methyl-2(5-methylhex-4-en-2-yl)cyclopropyl]-methanol;
- Aldehydes and ketones, e.g. anisaldehyde (4-methoxybenzaldehyde); alpha amyl cinnamic aldehyde (2-benzylideneheptanal); Georgywood^{™} (1-(1,2,8,8-tetramethyl-1,2,3,4,5,6,7,8-octahydronaphthalen-2-yl)ethanone); Hydroxycitronellal (7-hydroxy-3,7-dimethyloctanal); Iso E Super^{®} (1-(2,3,8,8-tetramethyl-1,2,3,4,5,6,7,8-octahydronaphthalen-2-yl)ethanone); Isoraldeine^{®} ((*E*)-3-methyl-4-(2,6,6-trimethylcyclohex-2-en-1-yl)but-3-en-2-one); Hedione^{®} (methyl 3-oxo-2-pentylcyclopentaneacetate); Nympheal (3-(4-isobutyl-2-methylphenyl)propanal); Mahonial (5,9-dimethyl-9-hydroxy-decen-4-al); maltol; methyl cedryl ketone; methylionone; verbenone; and/or vanillin;
- Ether and acetals, e.g. Ambrox^{®} (3a,6,6,9a-tetramethyl-2,4,5,5a,7,8,9,9b-octahydro-1*H*-benzo[*e*][1]benzofuran); geranyl methyl ether ((2*E*)-1-methoxy-3,7-dimethylocta-2,6-diene); rose oxide (4-methyl-2-(2-methylprop-1-en-1-yl)tetrahydro-2/7-pyran); and/ or Spirambrene^{®} (2',2',3,7,7-pentamethylspiro[bicyclo[4.1.0]heptane-2,5'-[1,3]dioxane]);
- Esters and lactones, e.g. benzyl acetate; cedryl acetate ((1*S*,6*R*,8a*R*)-1 ,4,4,6-tetramethyloctahydro-1*H*-5,8a-methanoazulen-6-yl acetate); γ-decalactone (6-pentyltetrahydro-2*H*-pyran-2-one); Helvetolide^{®} (2-(1-(3,3-dimethylcyclohexyl)ethoxy)-2-methylpropyl propionate); γ-undecalactone (5-heptyloxolan-2-one); and / or vetiveryl acetate ((4,8-dimethyl-2-propan-2-ylidene-3,3a,4,5,6,8a-hexahydro-1*H*-azulen-6-yl) acetate
- Macrocycles, e.g. Ambrettolide ((*Z*)-oxacycloheptadec-10-en-2-one); ethylene brassylate (1,4-dioxacycloheptadecane-5,17-dione); and / or Exaltolide^{®} (16-oxacyclohexadecan-1-one); and
- Heterocycles, e.g. isobutylquinoline (2-isobutylquinoline).

For example, the compound of formula (I) can be well combined with muguet compounds, as well as compounds with white florals, woody, musky, fruity (berry, plumy), spicy odour notes.

Those and other fragrance ingredients the compounds of formula (I) can be used in combination with are also described, for example, in "Perfume and Flavor Chemicals", S. Arctander, Ed., Vol. I & II, Allured Publishing Corporation, Carol Stream, USA, 2003 and include fragrance compounds of natural or synthetic origin and essential oils.

The compounds according to formula (I) may be used in a broad range of fragranced articles, e.g. in any field of fine and functional perfumery, such as perfumes, air care products, household products, laundry products, body care products and cosmetics. The compounds can be employed in widely varying amounts, depending upon the specific article and on the nature and quantity of other odorant ingredients. The proportion is typically from 0.0001 to 30 weight per cent of the article. In one embodiment, compounds of the present invention may be employed in a fabric softener in an amount of from 0.001 to 3 weight per cent. In another embodiment, compounds of the present invention may be used in fine perfumery in amounts from 0.01 to 20 weight per cent (e.g. up to about 10 weight per cent), more preferably between 0.01 and 5 weight per cent. However, these values are given only by way of example, since the experienced perfumer may also achieve effects or may create novel accords with lower or higher concentrations.

The compounds as described hereinabove may be employed in a consumer product base simply by directly mixing at least one compound of formula (I), or a fragrance composition with the consumer product base, or they may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the consumer product base.

Thus, the invention additionally provides a method of manufacturing a fragranced article, comprising the incorporation of a compound of formula (I), as a fragrance ingredient, either by directly admixing the compound to the consumer product base or by admixing a fragrance composition comprising a compound of formula (I), which may then be mixed with a consumer product base, using conventional techniques and methods. Through the addition of an olfactory acceptable amount of at least one compound of the present invention as hereinabove described the odour notes of a consumer product base will be improved, enhanced, or modified.

Thus, the invention furthermore provides a method for improving, enhancing or modifying a consumer product base by means of the addition thereto of an olfactorily acceptable amount of at least one compound of formula (I).

The invention also provides a consumer product comprising:
i) as odorant at least one compound of formula (I); and
ii) a consumer product base.

As used herein, 'consumer product base' means a composition for use as a consumer product to fulfil specific actions, such as cleaning, softening, and caring or the like. Examples of such products include fine perfumery, e.g. perfume and eau de toilette; fabric care, household products and personal care products such as laundry care detergents, rinse conditioner, personal cleansing composition, detergent for dishwasher, surface cleaner; laundry products, e.g. softener, bleach, detergent; body-care products, e.g. shampoo, shower gel; air care products (includes products that contain preferably volatile and usually pleasant-smelling compounds which advantageously can even in very small amounts mask unpleasant odours. Air fresheners for living areas contain, in particular, natural and synthetic essential oils such as pine needle oils, citrus oil, eucalyptus oil, lavender oil, and the like., in amounts for example of up to 50% by weight. As aerosols they tend to contain smaller amounts of such essential oils, by way of example less than 5% or less than 2% by weight, but additionally include compounds such as acetaldehyde (in particular, <0.5% by weight), isopropyl alcohol (in particular, <5% by weight), mineral oil (in particular, <5% by weight), and propellants.) and cosmetics.

In one particular embodiment the consumer product base is selected form fine perfumery, cosmetic products and personal care products, including deodorants, hair care products, soaps, and the like.

In a further particular embodiment the consumer product base is selected from fabric care products, including fabric softener, and home care products, including air fresheners, dish washers cleaning products and the like.

This list of products is given by way of illustration, and is not to be regarded as being in any way limiting.

The invention is now further described with reference to the following non-limiting examples. These examples are for the purpose of illustration only, and it is understood that variations and modifications can be made by one skilled in the art.

### EXAMPLES

### Example 1: 3-(3-propylphenyl)butanal

a) To a stirred solution of 3-bromobenzaldehyde (25 g, 135 mmol) in THF (250 mL) was added ethyl magnesium bromide (90 mL, 270 mmol, 3M in ether) dropwise at 0 °C. The reaction mixture was allowed to stir at 0 °C for 1 h. The reaction mixture was quenched with 1 N aq. HCl (pH-1-2) at 0°C, and extracted with EtOAc (2 x 250 mL). The combined organic layer was washed with brine (300 mL), dried over anhydrous sodium sulfate, filtered and concentrated under reduced pressure to afford the crude liquid. The crude product was purified over flash column chromatography eluting with 9-10 % EtOAc and pet ether as an eluent to afford 1-(3-bromophenyl) propan-1-ol as a colorless liquid (19.2 g, 66% yield).
b) To a stirred solution of 1-(3-bromophenyl) propan-1-ol (20 g, 93 mmol) in CH₂Cl₂ (200 mL), were added triethylsilane (223 mL, 1395 mmol) and boron trifluoride etherate (45.9 mL, 372 mmol) dropwise at -78 °C. The reaction was allowed to stir at room temperature for 16 h. The reaction mixture was quenched with ice water (100 mL), extracted with CH₂Cl₂ (2 x 200 mL). The combined organic layer was washed with brine (200 mL), dried over anhydrous sodium sulfate, filtered and concentrated under reduced pressure to afford the crude liquid. The crude product was purified by flash column chromatography eluting with 1% EtOAc in pet ether as an eluent to afford 1-bromo-3-propylbenzene as a colorless liquid (17 g, 90% yield).
c) To a stirred solution of 1-bromo-3-propylbenzene (17 g, 85 mmol) in THF (170 mL) was added n-butyl lithium (41.0 mL, 102 mmol, 2.5 M in hexane) at -78 °C. The reaction mixture was stirred at -78 °C for 1h and was added triethyl borate (17.44 mL, 102 mmol) at the same temperature. The reaction was allowed to stir at -78 °C for 1 h and at room temperature for 16 h. The reaction mixture was cooled to 0 °C, and quenched with aq. NH₄Cl (100 mL) and acidified with 1.5N aq. HCl (100 mL). The aqueous layer was extracted with EtOAc (2 x 200 mL). The combined organic layer was washed with brine (100 mL), dried over anhydrous sodium sulfate, filtered and concentrated under reduced pressure. The crude product was purified by flash column chromatography using 10% EtOAc and pet ether as an eluent to afford (3-propylphenyl) boronic acid as colorless liquid (9 g, 54% yield).
d) To a stirred solution of (3-propylphenyl) boronic acid (8 g, 48.8 mmol) in water (24 mL) and dioxane (68 mL) were added (E)-but-2-enal (5.66 mL, 68.3 mmol) and tripotassium phosphate (15.53 g, 73.2 mmol) in a sealed tube at room temperature. After degassing the reaction mixture for 10 min, chloro (1,5-cyclooctadiene) rhodium(I) dimer (0.722 g, 1.463 mmol) was added at room temperature and the reaction mixture was heated at 70 °C for 10 h. The reaction mixture was filtered through celite pad and washed with EtOAc (50 mL). The filtrate was washed with water (60 mL), dried over anhydrous sodium sulfate, filtered and concentrated under reduced pressure. The crude product was purified over reverse phase chromatography using 0.1 % formic acid in ACN to afford 3-(3-propylphenyl) butanal as colorless liquid (0.79 g, 8% yield). GC-MS (El, 70 eV): 190 (47, [M]^{+·}), 148 (37), 147 (95), 133 (33), 119 (64), 117 (40), 115 (36), 105 (83), 91 (100), 43 (38).

¹H NMR (400 MHz, CDCl₃, δ/ppm): 9.70 (t, J = 2.1 Hz, 1H), 7.26 - 7.17 (m, 1H), 7.05 - 7.01 (m, 3H), 3.33 (sxt, J = 7.1 Hz, 1H), 2.77 - 2.60 (m, 2H), 2.60 - 2.53 (m, 2H), 1.69 - 1.57 (m, 2H), 1.31 (d, J = 6.8 Hz, 3H), 0.94 (t, J = 7.3 Hz, 3H).

¹³C NMR (101 MHz, CDCl₃, δ/ppm): 202.0, 145.3, 143.0, 128.5, 127.0, 126.6, 123.9, 51.7, 38.1, 34.3, 24.6, 22.1, 13.8.

Odour description (10% solution in DPG): watery (ozonic), green (rubbery, linden blossom petal texture, bourgeonal), fatty (creamy), floral (muguet, white floral), aldehydic (sharp, metallic).

### Example 2: 3-(3-butylphenyl)butanal

a) To a stirred solution of 1-bromo-3-(bromomethyl)benzene (25 g, 100 mmol) in THF (250 mL) at 0 °C, was added dropwise Li₂CuCl₄ (32.0 mL, 3.20 mmol, 0.1 M in THF) followed by propyl magnesium bromide (180 mL, 180 mmol, 1 M in THF). The reaction mixture was allowed to stir at 0 °C for 1 h and at room temperature for 3 h. The reaction mixture was cooled to 0 °C, quenched with 20% w/w aq. acetic acid (100 mL) and water (200 mL). Organic layer was separated, dried over sodium sulfate and concentrated under vacuum. The crude product was purified twice over flash column chromatography eluting with neat pet ether as an eluent to afford 1-bromo-3-butylbenzene as a colorless liquid (10 g, 60% yield).
b) To a stirred solution of 1-bromo-3-butylbenzene (20 g, 94 mmol) in THF (200 mL) was added dropwise at -78 °C n-butyl lithium (45.0 mL, 113 mmol, 2.5 M in n-hexane). After stirring at -78 °C for 1 h, triethyl borate (16.44 g, 113 mmol) was added at -78 °C. Reaction was allowed to stir at -78 °C for 1 h and at room temperature for 4 h. The reaction mixture was cooled to 0 °C, quenched with aq. ammonium chloride solution (50 mL) and acidified with 1.5N aq. HCl (100 mL). The aqueous layer was extracted with EtOAc (2x 200 mL). The combined organic layers were washed with brine (100 mL), dried over anhydrous sodium sulphate, filtered and concentrated under vacuum. The crude product was purified by column chromatography using 18 % EtOAc and pet ether as an eluent to afford (3-butylphenyl)boronic acid as colorless liquid (12 g, 70% yield).
c) To a stirred solution of (3-butylphenyl)boronic acid (6 g, 33.7 mmol) in dioxane (47 mL) and water (14 mL) in a sealed tube were added (E)-but-2-enal 5 (3.54 g, 50.6 mmol) and tripotassium phosphate (10.73 g, 50.6 mmol) at room temperature. After degassing the reaction mixture for 10 min, chloro(1,5-cyclooctadiene)rhodium(I) dimer (0.499 g, 1.011 mmol) was added at room temperature. After stirring for 14 h at 70 °C, the reaction mixture was filtered through celite pad and washed with EtOAc (150 mL). The combined organic extracts were washed with water (200 mL), dried over anhydrous sodium sulphate, filtered and concentrated under vacuum. The crude product was purified by column chromatography eluting with 3-4% EtOAc in pet ether as an eluent followed by reverse phase chromatography using 0.1% HCOOH as an eluent to afford 3-(3-butylphenyl)butanal as a pale yellow liquid (0.21 g, 3% yield).

GC-MS (El, 70 eV): 204 (39, [M]^{+·}), 161 (37), 147 (80), 119 (79), 117 (50), 115 (39), 105 (94), 91 (100), 41 (45), 29 (34).

¹H NMR (400 MHz, CDCl₃, δ/ppm): 9.70 (t, J = 2.1 Hz, 1H), 7.27 - 7.12 (m, 1H), 7.05 - 7.01 (m, 3H), 3.37 - 3.27 (m, 1H), 2.77 - 2.54 (m, 4H), 1.65 - 1.52 (m, 2H), 1.43 - 1.22 (m, 5H), 1.02 - 0.84 (m, 3H).

¹³C NMR (101 MHz, CDCl₃, δ/ppm): 202.0, 145.3, 143.3, 128.5, 126.9, 126.6, 123.9, 51.8, 35.7, 34.3, 33.7, 22.4, 22.2, 13.9.

Odour description (10% solution in DPG): watery, fatty, fruity (koavone, allyl ionone), floral, aldehydic.

### Example 3: 3-(3-pentylphenyl)butanal

a) To a stirred suspension of magnesium (4.86 g, 200 mmol) in THF (100 mL), was added 1,2-dibromoethane (3.76 g, 20.01 mmol) and stirred at room temperature for 10 min. Then, a solution of 1-bromobutane (23.30 g, 170 mmol) in THF (30 mL) was added dropwise maintaining the reaction temperature at 25-35°C. After complete addition the reaction mixture was heated to 60 °C for 1 h. In a separate flask, Li₂CuCl₄ (32.0 ml, 3.20 mmol, 0.1 M in THF) was added dropwise at 0°C to a solution of 1-bromo-3-(bromomethyl)benzene (25 g, 100 mmol) in THF (120 mL) followed by the addition of the freshly prepared butyl magnesium bromide solution (see above). The reaction was allowed to stir at 0 °C for 1 h and at room temperature for 3 h. The reaction mixture was cooled to 0 °C, quenched with 20% aq. acetic acid (40 mL) and water (50 mL). The aqueous layer was extracted with MTBE (2x 300 mL). The combined organic extracts were washed with water (200 mL) and brine (200 mL), dried over anhydrous sodium sulfate, filtered and concentrated under reduced pressure. The crude product was purified by column chromatography (230-400 mesh size silica gel) using 0-2% EtOAc/Pet-ether as an eluent to afford 1-bromo-3-pentylbenzene as a pale-yellow liquid (11.5 g, 51% yield).
b) To a stirred solution of 1-bromo-3-pentylbenzene (15 g, 66.0 mmol) in THF (150 mL) was added n-butyl lithium (31.7 mL, 79 mmol) dropwise over a period of 20 min at -78 °C. The reaction mixture was stirred at -78 °C for 1 h. Triethyl borate (11.6 g, 79 mmol) was added at - 78 °C and stirring was continued at -78 °C for 1 h and at room temperature for 2 h. The reaction mixture was quenched at 0 °C with aq. NH₄Cl (50 mL) and acidified with 1.5N aq. HCl (100 mL). The aqueous layer was extracted with EtOAc (2x 200 mL), the combined organic extracts were washed with brine (200 mL), dried over anhydrous sodium sulfate, filtered and concentrated under reduced pressure. The crude product was purified by column chromatography (230-400 mesh size silica gel) using 10-20% EtOAc/Pet-ether as an eluent to afford (3-pentylphenyl) boronic acid as a white solid (8.6 g, 68% yield).
c) To a stirred solution of (3-pentylphenyl) boronic acid (7.5 g, 39.0 mmol) in dioxane (58 mL) and water (18 mL), were added (E)-but-2-enal 4 (2.74 g, 39.0 mmol) and K3PO4 (12.43 g, 58.6 mmol) at room temperature. The reaction mixture was degassed for 5 min. Chloro(1,5-cyclooctadiene) rhodium(I) dimer (0.578 g, 1.171 mmol) was added and stirring was continued at 60 °C for 16 h. The reaction mixture was filtered through celite pad and washed with EtOAc (100 mL). The combined organic extract was washed with water (200 mL), dried over anhydrous sodium sulfate, filtered, and concentrated under reduced pressure. The crude product was purified by column chromatography (1% EtOAc/ Pet-ether) and by reverse phase chromatography to afford 3-(3-pentylphenyl)butanal as a pale-yellow liquid (1.3 g, 15% yield). GC-MS (El, 70 eV): 218 (40, [M]^{+·}), 175 (35), 147 (98), 119 (89), 117 (55), 115 (36), 105 (87), 91 (100), 43 (68), 41 (37).

¹H NMR (400 MHz, CDCl₃, δ/ppm): 9.70 (t, J = 2.1 Hz, 1H), 7.24 - 7.18 (m, 1H), 7.04 - 7.01 (m, 3H), 3.33 (sxt, J = 7.1 Hz, 1H), 2.72 (dd, J = 2.0, 6.8 Hz, 1H), 2.77 - 2.55 (m, 3H), 1.64 - 1.57 (m, 2H), 1.38 - 1.28 (m, 7H), 0.92 - 0.87 (m, 3H).

¹³C NMR (101 MHz, CDCl₃, δ/ppm): 202.0, 145.3, 143.3, 128.5, 126.9, 126.5, 123.9, 51.7, 36.0, 34.3, 31.5, 31.2, 22.5, 22.1, 14.0.

Odour description (10% solution in DPG): watery, muguet, green, fatty, aldehydic, floral.

### Example 4: 3-(3-hexylphenyl)butanal

a) To a suspension of NaH (1.1 g, 27.4 mmol, 1.05 equiv, 60%) in THF (20 mL) was added dropwise a solution of ethyl 2-(diethoxyphosphoryl)acetate (6.14 g, 27.4 mmol, 1.05 equiv) in THF (15 mL). After stirring for 40 min at room temperature, a solution of 1-(3-hexylphenyl)ethan-1-one (5.33 g, 26.1 mmol, 1.0 equiv, was prepared according to Glass, R. S. et al., Sulfur Letters 1997, 2, 15-20) in THF (15 mL) was added dropwise (the temperature rose to 30°C). After completed addition, the cooling bath was removed and the reaction mixture was stirred for 2 hours at room temperature and for 18 hours at reflux. The reaction mixture was poured on ice cold 2M aq. HCl (15 mL), diluted with water (25 mL) and extracted with MTBE (50 mL). The organic layer was washed with 50 mL water and 2 x 50 mL brine. The organic layer was dried over MgSO₄, filtered and concentrated under reduced pressure to give crude ethyl 3-(3-hexylphenyl)but-2-enoate (6.88 g), which was used without further purification.
b) A suspension of crude ethyl 3-(3-hexylphenyl)but-2-enoate (6.88 g, 25.1 mmol, 1.0 equiv) and 0.10 g Pd/C in hexane (40 mL) was hydrogenated at ambient pressure and room temperature for 24 hours. Another portion of 0.04 g Pd/C was added and the reaction mixture was hydrogenated at ambient pressure and room temperature for another 24 hours. The reaction mixture was filtered and concentrated under reduced pressure to afford crude ethyl 3-(3-hexylphenyl)butanoate (6.57 g) as a clear, yellow liquid.
c) To a suspension of lithium aluminum hydride (0.72 g, 19.0 mmol, 0.8 equiv) in diethyl ether (40 mL) was added dropwise at 4°C a solution of crude ethyl 3-(3-hexylphenyl)butanoate (6.57 g, 23.8 mmol, 1.0 equiv) in diethyl ether (20 mL). After stirring for 2 hours at room temperature, the reaction still contained starting material. 0.18 g Lithium aluminium hydride powder was added and the reaction was stirred for another 30 min. The reaction mixture was quenched by slow and dropwise addition of 2M aq. NaOH (4.5 mL). After stirring for 10 min, one spatula of MgSO₄ was added and the mixture was stirred again for 5 min. Afterwards the reaction mixture was filtered and the solvent removed under reduced pressure to furnish crude 3-(3-hexylphenyl)butan-1-ol (5.64 g) as a clear, yellow liquid.
d) 3-(3-hexylphenyl)butan-1-ol (5.64 g, 24.1 mmol, 1.0 equiv), pyridinium chlorochromate (6.22 g, 28.9 mmol, 1.2 equiv) and CH₂Cl₂ (50 mL) were stirred for 1 hour at room temperature and filtered over silica gel (rinsed with heptane/MTBE 20:1). The concentrated crude product was purified by column chromatography (heptane / MTBE, 95:5) and Kugelrohr distillation (0.05 mbar) to furnish 3-(3-hexylphenyl)butanal (0.64 g, 11% yield over 4 steps) as a colorless liquid.

GC-MS (El, 70 eV): 232 (37, [M]^{+·}), 147 (100), 119 (90), 118 (39), 117 (63), 115 (39), 105 (81), 91 (96), 43 (61), 41 (45), 29 (41).

¹H NMR (400 MHz, CDCl₃, δ/ppm): 9.70 (t, J = 2.1 Hz, 1H), 7.26 - 7.18 (m, 1H), 7.05 - 7.01 (m, 3H), 3.33 (sxt, J = 7.0 Hz, 1H), 2.79 - 2.54 (m, 4H), 1.60 (quin, J = 7.5 Hz, 2H), 1.38 - 1.25 (m, 9H), 0.92 - 0.85 (m, 3H).

¹³C NMR (101 MHz, CDCl₃, δ/ppm): 202.1, 145.3, 143.4, 128.5, 126.9, 126.6, 123.9, 51.8, 36.0, 34.3, 31.7, 31.5, 29.0, 22.6, 22.2, 14.1.

Odour description (10% solution in DPG): green (metallic, hay), watery (marine), fatty, aldehydic (muguet).

### Example 5: 3-(3-heptylphenyl)butanal

a) To a solution of methyl 3-heptylbenzoate (19.5 g, 83.0 mmol, 1.0 equiv, prepared according to Ejiri, S. et al., Org. Lett. 2010, 12, 1692) in water (120 mL) and ethanol (60 mL) was added KOH (18.6 g, 332 mmol, 4 equiv). After refluxing for 1 hour, the reaction mixture was added to ice cold 2M aq. HCl (100 mL) and HCl conc. (20 mL) and extracted with MTBE. The organic layer was washed with water and brine. The organic layer was dried over MgSO4, filtered and concentrated under reduced pressure to afford crude 3-heptylbenzoic acid (16.8 g).
b) To a solution of 3-heptylbenzoic acid (16.8 g, 76.2 mmol, 1.0 equiv) in diethyl ether (160 mL) was added dropwise at -28°C methyl lithium (100 mL, 160 mmol, 2.1 equiv, 1.6M solution). After stirring for 1.5 h at room temperature, the reaction mixture as cautiously quenched by the dropwise addition of acetone (50 mL), poured on ice cold 2M aq. HCl (150 mL) and extracted with MTBE. The organic layer was washed with water and brine. The organic layer was dried over MgSO4, filtered and concentrated under reduced pressure. The crude was purified by fractional distillation over a 10 cm Vigreux column at high vacuum (0.03 mbar) to furnish 1-(3-heptylphenyl)ethan-1-one (4.56 g, 25% yield over 2 steps) as a colorless liquid.
c) To a suspension of NaH (0.85 g, 21.4 mmol, 1.05 equiv, 60%) in THF (20 mL) was added dropwise a solution of ethyl 2-(diethoxyphosphoryl)acetate (4.79 g, 21.4 mmol, 1.05 equiv) in THF (15 mL). After stirring for 40 min at room temperature, a solution of 1-(3-heptylphenyl)ethan-1-one (4.44 g, 20.3 mmol, 1.0 equiv) in THF (15 mL) was added dropwise. After completed addition, the cooling bath was removed and the reaction mixture was stirred for 2 hours at room temperature and for 18 hours at reflux. The reaction mixture was poured on ice cold 2M aq. HCl (15 mL), diluted with water (25 mL) and extracted with MTBE (50 mL). The organic layer was washed with 50 mL water and 2 x 50 mL brine. The organic layer was dried over MgSO₄, filtered and concentrated under reduced pressure to give crude ethyl 3-(3-heptylphenyl)but-2-enoate (5.7 g), which was used without further purification.
d) A suspension of crude ethyl ethyl 3-(3-heptylphenyl)but-2-enoate (5.7 g, 20 mmol, 1.0 equiv) and 0.09 g Pd/C in hexane (20 mL) was hydrogenated at ambient pressure and room temperature for 2 hours. The reaction mixture was filtered and concentrated under reduced pressure to afford crude ethyl 3-(3-heptylphenyl)butanoate (5.7 g) as a clear, yellow liquid.
e) To a suspension of lithium aluminum hydride (0.60 g, 15.7 mmol, 0.8 equiv) in diethyl ether (40 mL) was added dropwise at 4°C a solution of crude ethyl 3-(3-heptylphenyl)butanoate (5.71 g, 19.7 mmol, 1.0 equiv) in diethyl ether (20 mL). After stirring for 1 hour at room temperature, the reaction mixture was quenched by slow and dropwise addition of 2M aq. NaOH (3 mL). After stirring for 15 min, one spatula of MgSO₄ was added and the mixture was stirred again for 10 min. Afterwards the reaction mixture was filtered and the solvent removed under reduced pressure to furnish crude 3-(3-heptylphenyl)butan-1-ol (4.57 g) as a clear, yellow liquid.
f) 3-(3-heptylphenyl)butan-1-ol (4.57 g, 18.4 mmol, 1.0 equiv), pyridinium chlorochromate (4.76 g, 22.1 mmol, 1.2 equiv) and CH₂Cl₂ (50 mL) were stirred for 1 hour at room temperature and filtered over silica gel (rinsed with MTBE). The concentrated crude product was purified by column chromatography (heptane / MTBE, 95:5) and Kugelrohr distillation (0.05 mbar) to give 3-(3-heptylphenyl)butanal (1.18 g, 24% over 4 steps) as a colorless liquid.

GC-MS (El, 70 eV): 246 (34, [M]^{+·}), 147 (100), 119 (94), 118 (39), 117 (60), 105 (76), 91 (85), 43 (43), 41 (48), 29 (36).

¹H NMR (400 MHz, CDCl₃, δ/ppm): 9.70 (t, J = 2.1 Hz, 1H), 7.25 - 7.18 (m, 1H), 7.04 - 7.01 (m, 3H), 3.33 (sxt, J = 7.1 Hz, 1H), 2.80 - 2.52 (m, 4H), 1.64 - 1.56 (m, 2H), 1.36 - 1.23 (m, 11H), 0.91 - 0.85 (m, 3H).

¹³C NMR (101 MHz, CDCl₃, δ/ppm): 202.0, 145.3, 143.4, 128.5, 126.9, 126.6, 123.9, 51.7, 36.0, 34.3, 31.8, 31.5, 29.3, 29.2, 22.6, 22.1, 14.1.

Odour description (10% solution in DPG): marine (watery, oyster), green, floral.

### Example 6: Assessment of biodegradability

The results of the biodegradability assessment by the manometric respirometry test (OECD guideline for the testing of materials No. 301F, Paris 1992) are summarized in Table 2.

| **Compound of example** | **Compound name** | **Biodegradation in %** | **result** |
|---|---|---|---|
| 1 | 3-(3-propylphenyl)butanal | 81 | readily biodegradable |
| 2 | 3-(3-butylphenyl)butanal | 73 | inherently biodegradable |
| 3 | 3-(3-pentylphenyl)butanal | 81 | readily biodegradable |

The compounds of the present invention with linear substituents in meta-position relative to the aldehyde bearing functionality are biodegradable.

### Example 7: Comparison of GC threshold values

| **Compound of example** | **R1 (No. of C)** | **GC threshold (ng)** |
|---|---|---|
| 1 | *n*-propyl (3) | **4** |
| 2 | *n*-butyl (4) | **3.1** |
| 3 | *n*-pentyl (5) | **0.09** |
| *Comparative example a | 2-cyclopropylethyl (5) | 3.74 |
| *Comparative example b | 1-isopentyl (5) | 0.44 |
| Comparative example 5 | cyclopentyl (5) | 6 |
| GR-50-2775 | | |
| Comparative example 8 | neopentyl (5) | 0.73 |
| GR-88-0908 | | |
| 4 | *n*-hexyl (6) | **0.03** |
| Comparative example 6 | cyclopentylmethyl (6) | 7.9 |
| GR-50-2750 | | |
| *Comparative example c | 4-methylpentyl (6) | 0.34 |
| 5 | *n*-heptyl (7) | **0.73** |

*Comparative examples a to c are described in WO2014/205782.

For the compounds of the present invention with linear substituents in meta-position relative to the aldehyde bearing functionality, the GC threshold values are significantly lower than for the corresponding derivatives with branched, cyclic or unsaturated substituent with the same number of carbon atoms.

Therefore, it was surprisingly found, that the compounds of the present invention show a better odour per carbon ratio.

### Comparative example 1: 3-(3-ethylphenyl)butanal

a) To a suspension of NaH (1.45 g, 36.3 mmol, 1.05 equiv, 60%) in THF (30 mL) was added dropwise a solution of ethyl 2-(diethoxyphosphoryl)acetate (8.13 g, 36.3 mmol, 1.05 equiv) in THF (10 mL). After stirring for 1 hour at room temperature, a solution of 1-(3-ethylphenyl)ethan-1-one (5.12 g, 34.5 mmol, 1.0 equiv) in THF (10 mL) was added dropwise. After stirring for 1 hour at room temperature, the reaction mixture was poured on ice cold 2M aq. HCl (10 mL), diluted with water (30 mL) and extracted with MTBE (70 mL). The organic layer was washed with water and brine. The organic layer was dried over MgSO₄, filtered and concentrated under reduced pressure. The crude was purified by Kugelrohr distillation to furnish ethyl 3-(3-ethylphenyl)but-2-enoate (5.74 g) as a light yellow liquid.
b) A suspension of ethyl 3-(3-ethylphenyl)but-2-enoate (5.74 g, 26.3 mmol, 1.0 equiv) and 0.11 g Pd/C in hexane (50 mL) was hydrogenated at ambient pressure and room temperature for 21 hours. The reaction mixture was filtered and concentrated under reduced pressure to afford crude ethyl 3-(3-ethylphenyl)butanoate (5.66 g) as a colorless liquid.
c) To a suspension of lithium aluminum hydride (0.78 g, 20.6 mmol, 0.8 equiv) in diethyl ether (30 mL) was added dropwise at 0°C a solution of crude ethyl 3-(3-ethylphenyl)butanoate (5.66 g, 25.7 mmol, 1.0 equiv) in diethyl ether (20 mL). After stirring for 2 hours at room temperature, the reaction mixture was quenched by slow and dropwise addition of 2M aq. NaOH (4 mL). After stirring for 10 min, one spatula of MgSO₄ was added and the mixture was stirred for 10 min. Afterwards the reaction mixture was filtered and the solvent removed under reduced pressure to furnish crude 3-(3-ethylphenyl)butan-1-ol (4.25 g) as a colorless liquid.
d) 3-(3-ethylphenyl)butan-1-ol (4.25 g, 23.8 mmol, 1.0 equiv), pyridinium chlorochromate (6.17 g, 28.6 mmol, 1.2 equiv) and CH₂Cl₂ (40 mL) were stirred for 1 hour at room temperature and filtered over silica gel (rinsed with MTBE). The concentrated crude product was purified by column chromatography (heptane / MTBE, 95:5) and Kugelrohr distillation (0.05 mbar) to give 3-(3-ethylphenyl)butanal (1.25 g, 21% over 4 steps) as a colorless liquid.

GC-MS (El, 70 eV): 176 (46, [M]^{+·}), 147 (40), 134 (28), 133 (55), 119 (44), 117 (27), 115 (28), 105 (100), 91 (65), 29 (32).

¹H NMR (400 MHz, CDCl₃, δ/ppm): 9.71 (t, J = 2.1 Hz, 1H), 7.27 - 7.20 (m, 1H), 7.08 - 7.02 (m, 3H), 3.34 (sxt, J = 7.1 Hz, 1H), 2.81 - 2.54 (m, 4H), 1.32 (d, J = 7.1 Hz, 3H), 1.23 (t, J = 7.6 Hz, 3H).

¹³C NMR (101 MHz, CDCl₃, δ/ppm): 202.1, 145.4, 144.6, 128.6, 126.4, 126.0, 123.9, 51.7, 34.3, 28.9, 22.2, 15.6.

Odour description (10% solution in DPG): fatty, green (rubbery), aldehydic (muguet), marine, floral (florhydral).

### Comparative example 2: 3-(3-butylphenyl)propanal

a) To a degassed stirred solution of 1-bromo-3-butylbenzene (8.80 g, 41.3 mmol) in DMA (16 mL) were added zinc (8.10 g, 124 mmol), TBAI (4.58 g, 12.4 mmol), NiCl₂(DME) (1.82 g, 8.26 mmol) and pyridine-2,6-bis(carboximidamide) dihydrochloride (1.95 g, 8.26 mmol) at room temperature. A degassed solution of (3-bromopropoxy)(tert-butyl)dimethylsilane (10.5 g, 41.3 mmol) in DMA (16 mL) was added to the above reaction mixture and the reaction was allowed to stir at 90 °C for 10 h. The reaction mixture was diluted with MTBE (100 mL), filtered through celite pad, washed with MTBE (100 mL). Organic layer was washed with ice water, dried over sodium sulfate and concentrated under vacuum. The crude was purified by column chromatography eluting with neat pet ether as an eluent to afford tert-butyl(3-(3-butylphenyl)propoxy)dimethylsilane as a colorless liquid (4.7 g, 36% yield).
b) To a stirred solution of tert-butyl(3-(3-butylphenyl)propoxy)dimethylsilane (3.7 g, 12.1 mmol) in THF (37 mL) was added TBAF (24.1 mL, 24.1 mmol) at 0 °C. After stirring for 16 h at room temperature, the reaction mixture was quenched with water (100 mL), concentrated under vacuum and extracted with EtOAc (150 mL). Organic layer was dried over sodium sulfate and concentrated under reduced pressure. The crude was purified by column chromatography eluting with 2% EtOAc in pet ether as an eluent to afford 3-(3-butylphenyl)propan-1-ol as a colorless liquid (2.0 g, 82% yield).
c) To a stirred solution of 3-(3-butylphenyl)propan-1-ol 4 (2.0 g, 10.40 mmol) in CH₂Cl₂ (20 mL) was added Dess-Martin periodinane (6.62 g, 15.6 mmol) at 0 °C. After stirring for 2 hours at room temperature, the reaction mixture was quenched with aq. NaHCOs (50 mL). The suspension was filtered through a celite pad and washed with CH₂Cl₂ (50 mL). The organic layer was separated, dried over sodium sulfate and concentrated under vacuum. The crude was purified by column chromatography eluting with 1% EtOAc in pet ether as an eluent followed by reverse phase chromatography using 0.1% HCOOH/MeCN to afford 3-(3-butylphenyl)propanal as a colorless liquid (1.16 g, 56% yield).

GC-MS (El, 70 eV): 190 (31, [M]^{+·}), 148 (20), 134 (21), 120 (18), 117 (22), 115 (20), 105 (100), 104 (31), 92 (39), 91 (59).

¹H NMR (400 MHz, CDCl₃, δ/ppm): 9.79 (t, J = 1.5 Hz, 1H), 7.22 - 7.17 (m, 1H), 7.03 - 6.97 (m, 3H), 2.94 - 2.90 (m, 2H), 2.77 - 2.73 (m, 2H), 2.60 - 2.55 (m, 2H), 1.62 - 1.54 (m, 2H), 1.39 - 1.30 (m, 2H), 0.92 (t, J = 7.3 Hz, 3H).

¹³C NMR (101 MHz, CDCl₃, δ/ppm): 201.7, 143.2, 140.1, 128.4, 128.4, 126.3, 125.4, 45.2, 35.5, 33.6, 28.0, 22.3, 13.9.

Odour description (10% solution in DPG): green (green, apple) fatty (waxy), floral (muguet), aldehydic (metallic, sharp).

### Comparative example 3: 3-(3-pentylphenyl)propanal

a) To a stirred solution of 1-bromo-3-pentylbenzene (5.0 g, 22.0 mmol) in degassed DMA (50 mL) was added zinc (4.32 g, 66.0 mmol), TBAI (2.44 g, 6.60 mmol) and NiCl₂(DME) (0.97 g, 4.40 mmol). The reaction mixture was then evacuated and backfilled with nitrogen (3 times) with the help of a 2-way adaptor and degassed for 10 min. Then, pyridine-2,6-bis(carboximidamide) dihydrochloride (0.21 g, 0.88 mmol) was added and purged with nitrogen for 5 min. A degassed solution of 3-bromopropan-1-ol (3.06 g, 22.0 mmol) in DMA (50 mL) was added to the above reaction mixture. After stirring for 10 h at 90 °C for 10 h, the reaction mixture was quenched with water (50 mL), filtered through a celite bed. The resulting solution was extracted with ethyl acetate (2x 100 mL), washed with water (3x 50 mL)), brine (50 mL), dried over anhydrous sodium sulfate, filtered, and concentrated under reduced pressure. The crude was purified by column chromatography (0-3% ethyl acetate in pet ether) to afford 3-(3-pentylphenyl) propan-1-ol (1.2 g, 26% yield).
b) To a stirred solution of 3-(3-pentylphenyl) propan-1-ol (2.3 g, 11.2 mmol) in CH₂Cl₂ (70 mL) was added Dess-Martin periodinane (6.15 g, 14.5 mmol) at 0 °C. The reaction was stirred at room temperature for 2 h. The reaction was quenched with 10% aq. sodium bicarbonate solution (25 mL), and filtered through a celite bed. The filtrate was washed with CH₂Cl₂ (2x 75 ml) and the combined organic extracts were dried over anhydrous sodium sulfate, filtered and concentrated under vacuum. The crude was purified by column chromatography (0-1% of ethyl acetate in pet ether) to afford 3-(3-pentylphenyl)propanal as a colorless liquid (1.32 g, 58% yield).

GC-MS (El, 70 eV): 204 (21, [M]^{+·}), 148 (21), 133 (21), 117 (23), 115 (19), 105 (100), 104 (36), 92 (40), 91 (57), 29 (20).

¹H NMR (400 MHz, CDCl₃, δ/ppm): 9.82 (t, J = 1.6 Hz, 1H), 7.20 (t, J = 7.2 Hz, 1H), 7.04 - 6.98 (m, 3H), 2.95 - 2.90 (m, 2H), 2.80 - 2.74 (m, 2H), 2.59 - 2.54 (m, 2H), 1.65 - 1.55 (m, 2H), 1.39 - 1.25 (m, 4H), 0.92 - 0.86 (m, 3H)

¹³C NMR (101 MHz, CDCl₃, δ/ppm): 201.7, 143.3, 140.2, 128.4, 128.4, 126.4, 125.5, 45.3, 35.9, 31.5, 31.2, 28.1, 22.5, 14.0.

Odour description (10% solution in DPG): green (green apple), aldehydic (metallic, hot iron), floral (muguet).

### Comparative example 4: 3-(3-hexylphenyl)propanal

To a stirred solution of 3-(3-hexylphenyl)propan-1-ol (5.8 g, 26.3 mmol, prepared according to WO2022237852) in CH₂Cl₂ (58 mL) was added PCC (8.51 g, 39.5 mmol) at 0 °C. After stirring for 4 h at room temperature, the reaction mixture was concentrated under vacuum. The black residue was triturated with MTBE (2 x 100 mL) and filtered through a celite pad. The filtrate was concentrated under reduced pressure. The crude was purified by reverse phase chromatography eluting with 0.1% HCOOH/MeCN to afford 3-(3-hexylphenyl)propanal as a colourless liquid (2.9 g, 50% yield).

GC-MS (El, 70 eV): 218 (21, [M]^{+·}), 133 (23), 120 (22), 117 (27), 115 (19), 105 (100), 104 (41), 92 (45), 91 (54), 43 (22).

¹H NMR (400 MHz, CDCl₃, δ/ppm): 9.81 (t, J = 1.5 Hz, 1H), 7.19 (t, J = 7.2 Hz, 1H), 7.04 - 6.98 (m, 3H), 2.95 - 2.90 (m, 2H), 2.78 - 2.71 (m, 2H), 2.59 - 2.54 (m, 2H), 1.63 - 1.55 (m, 2H), 1.37 - 1.26 (m, 6H), 0.91 - 0.85 (m, 3H).

¹³C NMR (101 MHz, CDCl₃, δ/ppm): 201.7, 143.3, 140.1, 128.4, 128.4, 126.3, 125.4, 45.3, 35.9, 31.7, 31.5, 29.0, 28.1, 22.6, 14.1.

Odour description (10% solution in DPG): aldehydic (metallic, sharp, thin, hot iron).

### Comparative example 5: 3-(3-cyclopentylphenyl)butanal

a) To a stirred solution of 1,3-dibromobenzene (10 g, 42.4 mmol) in a mixture of 1,4-dioxane (90 mL) and water (10 mL) was added cyclopent-1-en-1-ylboronic acid (3.80 g, 33.9 mmol) and K₂CO₃ (14.7 g, 106 mmol) at room temperature. The reaction mixture was degassed with nitrogen for 5 min, and PdCl₂(dppf)-CH₂Cl₂ (1.73 g, 2.12 mmol) was added. After stirring for 3 hours at 95°C, the reaction mixture was diluted with ethyl acetate (100 mL), washed with water (80 mL) and brine solution (80 mL). The organic layer was dried over anhydrous sodium sulfate, filtered and concentrated under reduced pressure. The crude was purified by column chromatography (100% petrol ether) to afford 1-bromo-3-(cyclopent-1-en-1-yl)benzene (7.52 g, 52 % yield) as a colorless liquid.
b) To a solution of 1-bromo-3-(cyclopent-1-en-1-yl)benzene (5.6 g, 25.10 mmol) in ethyl acetate (70 mL) was added platinum(IV) oxide (0.342 g, 1.506 mmol) under nitrogen atmosphere at room temperature. After stirring for 16 hours under 1 atm of hydrogen pressure, the reaction mixture was filtered through a celite pad, which was washed with ethyl acetate (100 mL). The filtrate was concentrated under reduced pressure. The crude was purified by column chromatography (100% petrol ether) to afford 1-bromo-3-cyclopentylbenzene (5.1 g, 57% yield) as a colorless liquid.
c) To a stirred solution of 1-bromo-3-cyclopentylbenzene (5.1 g, 22.7 mmol) in THF (80 mL) was added dropwise n-butyllithium (10.9 mL, 27.2 mmol, 2.5M in hexane) over 45 min at -78°C. After stirring the reaction mixture for 1 hour at that temperature, triethyl borate (3.97 g, 27.2 mmol) was added at -78 °C and continued the stirring was continued for another hour at the same temperature. The reaction mixture was warmed up to 0 °C and quenched with aq. NH₄Cl solution (30 mL) and aq. 1.5N HCl (20 mL). The mixture was extracted with EtOAc (2x 80 mL). The combined organic extracts were washed with brine (50 mL), dried over anhydrous sodium sulfate, filtered and concentrated under reduced pressure. The crude was purified by column chromatography (0-10% EtOAc in petrol ether) to afford (3-cyclopentylphenyl)boronic acid (2.5 g, 58% yield) as a colorless liquid.
d) To a stirred solution of (3-cyclopentylphenyl)boronic acid (2.4 g, 12.6 mmol) in a mixture of 1,4-dioxane (25 mL) and water (5 mL) was added (E)-but-2-enal (1.06 g, 15.2 mmol) and tripotassium phosphate (2.68 g, 12.6 mmol) at room temperature. After degassing the mixture with nitrogen for 10 min, chloro(1,5-cyclooctadiene)rhodium(I) dimer (3.11 mg, 6.31 µmol) was added. After stirring for 12 hours at 55 °C, the reaction mixture was diluted with ethyl acetate (100 mL), washed with water (80 mL) and brine solution (80 mL). The organic layer was dried over anhydrous sodium sulfate, filtered and concentrated under reduced pressure. The crude was purified by column chromatography (0-3% MTBE in petrol ether) to afford 3-(3-cyclopentylphenyl)butanal (1.04 g, 37% yield) as a colorless liquid.

¹H NMR (400 MHz, CDCl₃, δ/ppm): 9.70 (t, J = 2.0 Hz, 1H), 7.29 - 7.15 (m, 1H), 7.12 - 7.06 (m, 2H), 7.05 - 6.99 (m, 1H), 3.38 - 3.28 (m, 1H), 3.03 - 2.90 (m, 1H), 2.77 - 2.60 (m, 2H), 2.11 - 2.00 (m, 2H), 1.84 - 1.75 (m, 2H), 1.73 - 1.53 (m, 4H), 1.31 (d, J = 7.0 Hz, 3H).

¹³C NMR (101 MHz, CDCl₃, δ/ppm): 202.0, 146.9, 145.3, 128.5, 125.7, 125.2, 123.9, 51.7, 45.9, 34.6, 34.3, 25.5, 22.1.

Odour description (10% solution in DPG): green, floral, rubbery, muguet.

### Comparative example 6: 3-(3-(cyclopentylmethyl)phenyl)butanal

a) To a stirred solution of 1-bromo-3-(bromomethyl)benzene (15 g, 60.0 mmol) in THF (150 mL) was added Li₂CuCl₄ (1M in THF) (18.0 mL, 1.80 mmol) and followed by cyclopentylmagnesium bromide (33.0 mL, 66.0 mmol, 2M in THF) at 0 °C. It was stirred for 3h at rt. The progress of the reaction was monitored by TLC. Upon completion of the reaction as confirmed by TLC. The reaction mixture was cooled to 0 °C and quenched with 50% acetic acid (200 mL) and extracted with ethyl acetate (2x 250 mL). The organic layer was washed with water (100 mL), dried over sodium sulfate, filtered, and concentrated under reduced pressure. The crude was purified by column chromatography (100% petrol ether) to afford 1-bromo-3-(cyclopentylmethyl)benzene (6.1 g, 38 % yield)
b) To a stirred solution of 1-bromo-3-(cyclopentylmethyl)benzene (6.1 g, 25.5 mmol) in THF (60 mL) was added dropwise at -78 °C n-butyl lithium (12.2 mL, 30.6 mmol, 2.5M in hexane). After stirring for 1 hour at same temperature, triethyl borate (5.2 mL, 30.6 mmol) was added at -78 °C and stirring was continued for another 2 hours. The reaction mixture was quenched with aq. NH₄Cl solution (50 mL) and aq. 1.5N HCl (50 mL) at 0 °C, and extracted with EtOAc (2x 75 mL). The combined organic extracts were washed with brine (50 mL), dried over sodium sulfate, filtered, and concentrated under reduced pressure. The crude was purified by column chromatography (0-30% EtOAc in petrol ether) to furnish (3-(cyclopentylmethyl)phenyl)boronic acid (3.5 g, 61 % yield) as an off white gummy solid.
c) To a stirred solution of (3-(cyclopentylmethyl)phenyl)boronic acid (2.9 g, 14.2 mmol) in 1,4-dioxane (23 mL) and water (3.5 mL) was added (E)-but-2-enal (1.12 g, 17.1 mmol), tripotassium phosphate (3.02 g, 14.2 mmol) and (1Z,5Z)-cycloocta-1,5-diene (0.138 g, 1.28 mmol) at room temperature. After degassing the reaction mixture with nitrogen for 5 minutes, chloro(1,5-cyclooctadiene)rhodium(I) dimer (3.50 mg, 7.11 µmol) was added. After heating at 55 °C for 12 hours, the reaction mixture was diluted with ethyl acetate (50 mL), washed with water (25 mL), brine solution (25 mL). The organic phase was dried over sodium sulfate, filtered, and concentrated. The residue was purified by column chromatography (0-4% EtOAc in petrol ether) to afford 3-(3-(cyclopentylmethyl)phenyl)butanal (1.36 g, 41 % yield) as a colorless liquid. ¹H NMR (400 MHz, CDCl₃, δ/ppm): 9.69 (t, J = 2.1 Hz, 1H), 7.21 (t, J = 7.0 Hz, 1H), 7.05 - 6.99 (m, 3H), 3.37 - 3.27 (m, 1H), 2.76 - 2.55 (m, 4H), 2.12 - 2.00 (m, 1H), 1.73 - 1.45 (m, 6H), 1.31 (d, J = 7.0 Hz, 3H), 1.24 - 1.10 (m, 2H).

¹³C NMR (101 MHz, CDCl₃, δ/ppm): 202.0, 145.2, 142.7, 128.4, 127.3, 126.9, 123.8, 51.7, 42.1, 41.9, 34.3, 32.4, 24.9, 22.1.

Odour description (10% solution in DPG): aldehydic, green, rubbery, watery, marine, melon.

### Comparative example 7: 3-(3-isobutylphenyl)butanal

a) To a suspension of lithium (717 mg, 103 mmol, 2.0 equiv) in THF (60 mL) at -40°C was added a solution of 1-chloro-3-(2-methylallyl)benzene (8.4 g, 50.4 mmol, 1.0 equiv) and trimethyl borate (10.5 g, 101 mmol, 2.0 equiv) in THF (30 mL) during 30 minutes. After the reaction was initiated, the reaction mixture was allowed to stir at -20°C for 4 hours and at room temperature overnight. The reaction mixture was cooled to 0°C, aq. 6M HCl solution (8.5 mL, 51.0 mmol, 1.0 equiv) was carefully added to the mixture, and the resulting mixture was allowed to stir at 10°C for 30 minutes. The reaction mixture was extracted with MTBE (3 x 100 mL). The combined organic extracts were dried over MgSO₄, filtered and the solvent was removed by rotary evaporation. The obtained residue was diluted with water (80 mL) and the mixture was heated to reflux for 1 hour. After the reaction mixture was cooled to 0°C, a white solid precipitated, which was filtered off to obtain crude (3-(2-methylallyl)phenyl)boronic acid. Potassium carbonate (5.80 g, 42.0 mmol, 1.0 equiv) and cesium carbonate (340 mg, 1.0 mmol, 2 mol%) were added to a mixture of the above crude (3-(2-methylallyl)phenyl)boronic acid (1.2 equiv), (E)-but-2-enal (3.0 g, 42.0 mmol, 1.0 equiv), palladium acetate (118.0 mg, 0.53 mmol, 1.3 mol%), and triphenylphosphine (550 mg, 2.1 mmol, 5 mol%) in toluene (90 mL) and chloroform (0.45 mL) under Ar. The resulting mixture was stirred under Ar at 90°C for 16 hours. After the reaction mixture had cooled to room temperature, water (200 mL) was added and the mixture was extracted with MTBE (3x 100 mL). The combined organic extracts were dried over MgSO₄, filtered and the solvent was removed by rotary evaporation. The residue was purified by flash chromatography (heptane: MTBE=9:1) to give 3-(3-(2-methylallyl)phenyl)butanal (1.5 g, 7.3 mmol, 15% yield) as a colorless liquid.

¹H NMR (300 MHz, CDCl₃, δ/ppm): 9.70 (t, J = 2.2 Hz,1H), 7.31 - 7.16 (m, 1H), 7.15 - 6.96 (m, 3H), 4.81 (s, 1H), 4.72 (s, 1H), 3.45 - 3.22 (m, 3H), 2.82 - 2.55 (m, 2H), 1.67 (s, 3H), 1.31 (d, J = 6.9 Hz, 3H).

¹³C NMR (75 MHz, CDCl₃, δ/ppm) 202.07(d), 145.53(s), 145.10(s), 140.21(s), 128.69(d), 127.51(d), 127.25(d), 124.51(d), 112.09(t), 51.84(t), 44.17(t), 34.38(d), 22.26(q), 22.18(q). GC/MS (El): m/z (%): 202 (76) [M^{+·}], 187 (34), 169 (20), 159 (70), 147 (91), 131 (82), 117 (100), 105 (55), 91 (66), 77 (14), 55 (21).

Odour description (10% solution in DPG): floral, green, muguet, rubbery.

b) A suspension of 3-(3-(2-methylallyl)phenyl)butanal (506 mg, 2.5 mmol, 1.0 equiv) and 10% Pd/C (50 mg) in ethyl acetate (20 mL) was stirred under an atmospheric H₂ pressure for 4 hours. The reaction mixture was filtered and the solvent was removed by rotary evaporation. The residue was purified by flash chromatography (heptane: MTBE=9:1) and by Kugelrohr distillation (0.10 mbar, 160°C) to give 3-(3-isobutylphenyl)butanal (464 mg, 2.3 mmol, 91% yield) as a colorless liquid.

¹H NMR (300 MHz, CDCl₃, δ/ppm): 9.69 (t, J = 2.0 Hz,1H), 7.29 - 7.15 (m, 1H), 7.10 - 6.93 (m, 3H), 3.47 - 3.21 (m, 1H), 2.81 - 2.56 (m, 2H), 2.45 (d, J=7.1Hz, 2H), 1.99 - 1.74 (m, 1H), 1.31 (d, J = 7.0 Hz,3H), 0.89 (d, J = 6.6 Hz,6H).

¹³C NMR (75 MHz, CDCl₃, δ/ppm): 202.12(d), 145.27(s), 142.17(s), 128.48(d), 127.75(d), 127.44(d), 124.07(d), 51.89(t), 45.56(t), 34.41(d), 30.34(d), 22.48(q), 22.28(q).

GC/MS (El): m/z (%): 204 (71) [M^{+·}], 189 (11), 161 (46), 147 (61), 133 (25), 119 (100), 105 (44), 91 (63), 77 (12), 65 (8), 57 (20), 41 (24).

Odour description (10% solution in DPG): rubbery, aldehydic, hot iron, floral, muguet.

### Comparative example 8: 3-(3-neopentylphenyl)butanal

a) To a solution of 1-bromo-3-neopentylbenzene (8.4 g, 37.0 mmol, 1.0 equiv) in dioxane (74 mL) was added Pd₂(dba)₃ (0.68 g, 2 mol%), 2-dicyclohexylphosphino-2',4',6'-triisopropyl-biphenyl (XPhos) (0.71 g, 4 mol%), KOAc (10.9 g, 111 mmol, 3.0 equiv) and 4,4,4',4',5,5,5',5'-octamethyl-2,2'-bi(1,3,2-dioxaborolane) (11.3 g, 44.4 mmol, 1.2 equiv). The reaction mixture was heated to 110 °C for 20 hours under nitrogen. After filtration over celite and washing of the filter cake with EtOAc (100 mL), the organic phase was poured on ice-cold water (100 mL) and stirred vigorously. The phases were separated and the aqueous phase was extracted with EtOAc (2x 100 mL). The combined organic extracts were washed with water (100 mL), brine (100 mL), dried over NaSO₄, filtered and concentrated under reduced pressure. The crude was purified by column chromatography (pentane/EtOAc, 39:1) to afford 4,4,5,5-tetramethyl-2-(3-neopentylphenyl)-1,3,2-dioxaborolane (8.1 g, 80% yield) as a yellow oil. ¹H NMR (400 MHz, CDCl₃, δ/ppm): 7.68 - 7.61 (m, 1H), 7.59 - 7.53 (m, 1H), 7.29 - 7.21 (m, 2H), 2.50 (s, 2H), 1.34 (s, 12H), 0.91 (s, 9H). ¹³C NMR (101 MHz, CDCl₃, δ/ppm): 139.0, 136.7, 133.4, 132.2, 127.0, 83.6, 50.0, 31.7, 29.4, 24.9.
b) To a suspension of 4,4,5,5-tetramethyl-2-(3-neopentylphenyl)-1,3,2-dioxaborolane (8.1 g, 29.5 mmol, 1.0 equiv), (E)-but-2-enal (2.09 g, 29.5 mmol, 1.0 equiv) and Pd(OAc)₂ (0.17 g, 2.5 mol%) in toluene (55 mL) and chloroform (0.3 mL, 200:1 ratio) was added K₂CO₃ (8.25 g, 59.1 mmol, 2.0 equiv), Cs₂CO₃ (0.49 g, 5 mol%) and PPh₃ (0.78 g, 10 mol%). The reaction mixture was heated to 90 °C over night, filtered over celite and the filter cake was washed with MTBE (100 mL). The filtrate was diluted wit MTBE (100 mL) and ice-cold water (100 mL). The phases were separated and the aqueous phase was extracted with MTBE (2x 100 mL). The combined organic extracts were washed with water (100 mL), brine (100 mL), dried over NaSO₄, filtered and concentrated under reduced pressure. The crude was purified twice by column chromatography (pentane/EtOAc, 39:1) and Kugelrohr distilled (120-135°C / 0.2 mbar) to afford 3-(3-neopentylphenyl)butanal (3.0 g, 39% yield) as a colorless oil.

¹H NMR (400 MHz, CDCl₃, δ/ppm): 9.70 (s, 1H), 7.22 - 7.18 (m, 1H), 7.05 (d, J = 7.8 Hz, 1H), 6.98 (d, J = 7.6 Hz, 2H), 3.36 - 3.30 (m, 1H), 2.77 - 2.70 (m, 1H), 2.67 - 2.60 (m, 1H), 2.47 (s, 2H), 1.31 (d, J = 7.1 Hz, 3H), 0.89 (s, 9H) ppm.

¹³C NMR (101 MHz, CDCl₃, δ/ppm): 201.9, 144.6, 140.1, 129.0, 128.7, 127.9, 124.1,51.8,50.2, 34.3, 31.7, 29.4, 22.2 ppm.

Odour description (10% solution in DPG): aldehydic hot iron,metallic, green rubbery fatty.

### Example 9: Perfumery Example

| **CAS** | **Chemical Name (IUPAC)** | **parts** |
|---|---|---|
| 112-45-8 | 10-Undecenal | 2.000 |
| 24817-51-4 | 2-Phenylethyl 2-Methylbutanoate | 30.000 |
| 140-11-4 | Benzyl Acetate | 30.000 |
| 106-22-9 | 3,7-Dimethyl-6-Octen-1-Ol | 60.000 |
| 23696-85-7 | 1-(2,6,6-Trimethyl-1,3-Cyclohexadiene-1-YI)-2-Butene-1-One | 2.000 |
| 93-92-5 | 1-Phenylethyl Acetate | 20.000 |
| 24851-98-7 | Methyl 3-Oxo-2-Pentylcyclopentaneacetate | 110.000 |
| 928-96-1 | 3-Hexen-1-OI (Z)- | 4.000 |
| 3681-71-8 | Cis-3-Hexenyl Acetate | 4.000 |
| 61444-38-0 | Cis-3-Hexenyl Cis-3-Hexenoate | 8.000 |
| 41519-23-7 | Cis-3-Hexenyl 2-Methylpropanoate | 8.000 |
| 65405-77-8 | Cis-3-Hexenyl 2-Hydroxybenzoate | 40.000 |
| 142-92-7 | Hexyl Acetate | 40.000 |
| | Benzopyrrole (1 % Solution In Triethyl Citrate) | 70.000 |
| 79-77-6 | 4-(2,6,6-Trimethyl-1-Cyclohexen-1-YI)-3-Buten-2-One | 60.000 |
| 488-10-8 | 3-Methyl-2-(Pent-2-En-1-Yl)Cyclopent-2-Enone | 10.000 |
| 873888-84-7 | (4Z)-Hept-4-En-2-YI Salicylate | 40.000 |
| 8008-56-8 | Oils, Lemon | 60.000 |
| 1365-19-1 | 2-Methyl-2-Vinyl-5-(1-Hydroxy-1-Methylethyl)Tetra-Hydrofuran | 10.000 |
| 78-70-6 | 3,7-Dimethyl-1,6-Octadien-3-Ol | 160.000 |
| 134-20-3 | Methyl 2-Aminobenzoate | 10.000 |
| 119-36-8 | Methyl 2-Hydroxybenzoate | 4.000 |
| 16409-43-1 | 4-Methyl-2-(2-Methyl-1-Propenyl)Tetrahydro-2h-Pyran | 8.000 |
| 113486-29-6 | 3-Methyl-2,4-Nonadione | 10.000 |
| | **total** | **800.000** |

To the fragrance compositions mentioned above 20% DPG were added (1000.000 in total) to form the reference.
a) 20% DPG of the reference were replaced by + 0.5% 3-(3-pentylphenyl)butanal (compound of example 3) + 19.5 % DPG
b) 20% DPG of the reference were replaced by + 1.5% 3-(3-pentylphenyl)butanal (compound of example 3) + 18.5 % DPG
c) 20% DPG of the reference were replaced by + 1.5% Nympheal + 18.5 % DPG
d) 20% DPG of the reference were replaced by 0.5% 3-(3-pentylphenyl)butanal (compound of example 3) + 1.5% Nympheal + 18.0 % DPG

The reference and samples a) to d) were added in 0.5% to a shampoo base.

The reference is a floral accord to be applied @ 0.5% in shampoo. It develops a floral tropical accord around the Ashok Indian flower concept.

In sample a) the top note is much more watery floral with a clear enhanced bloom in use in cabin. In sample b) the top note is much more watery floral with a clear enhanced bloom and performance in use in cabin, the compound of the present invention compares in performance to Nympheal (3-(4-isobutyl-2-methylphenyl)propanal) in bloom shampoo in cabins. In sample c), the top note benefits from the combination of the watery floral contribution from the compound of the present invention and the rich creamy floral muguet contribution from Nympheal, with a clear enhanced bloom and performance in use in cabin. The compound of example 3 combines well with Nympheal to enrich the floral creaminess and opens to additional watery marine aspects.

### Example 10: Application in liquid detergent

a) Sample preparation 0.2% by weight of 3-(3-ethylphenyl)butanal, (compound of comparative example 1, sample A), 3-(3-pentylphenyl)butanal (compound of example 3, sample B) or Nympheal (3-(4-isobutyl-2-methylphenyl)propanal, sample C) were incorporated respectively into unperfumed liquid detergent base by magnetic stirring at room temperature for 24 h.
b) Washtest and sensory evaluation

A 40°C machine wash cycle was performed using 55 g of the above prepared liquid detergent samples A-C and odor-neutral cotton/elastan mixed fabric T-shirts. The wet and line-dried fabric (1 and 3 days) was assessed by a panel of 4-6 experts with regard to odor intensity and quality. The odor intensity was recorded on an intensity scale of 0 (odorless) to 5 (extremely strong). As can be seen from Table 1 below, 3-(3-pentylphenyl)butanal (sample B) does perform equally well as Nympheal (3-(4-isobutyl-2-methylphenyl)propanal, sample C) across the different wet and dry down stages. In contrast, 3-(3-ethylphenyl)butanal (sample A) is significantly weaker than sample B and C at dry stages (1 and 3 days).

**Table 1**

| Sample | Wet | 1 day | 3 days |
|---|---|---|---|
| 3-(3-ethylphenyl)butanal (**A**) | 2.5 | 2.4 | 2.1 |
| 3-(3-pentylphenyl)butanal (**B**) | 2.8 | 3.3 | 3.8 |
| Nympheal (**C**) | 3.1 | 3.3 | 3.3 |

The performance of the compound of the present invention is comparable to Nympheal, while the comparative example 1 (with a C2 side chain) lacks substantivity.

### Example 11: Activation of OR2J2 by the compounds of the present invention 3-(3-pentylphenyl)butanal receptor activation

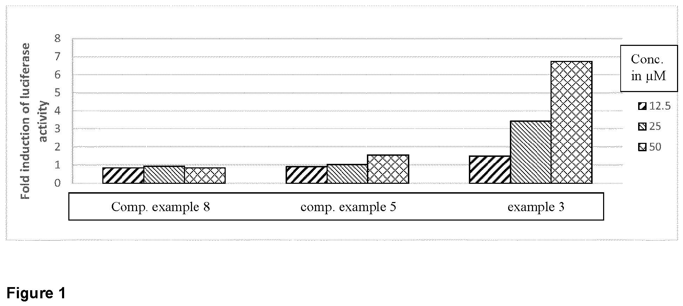

A library of all human class II olfactory receptors was screened for activation by 3-(3-pentylphenyl)butanal. OR2J2 was found to be activated by this compound. Figure 1 shows activation of OR2J2 by compounds of comparative example 8, comparative example 5 and example 3. According to the receptor activation data in Fig. 1, it can be concluded that compounds of the present invention do significantly activate the OR2J2 receptor in contrast to the prior art compounds.

To create the expression plasmid for human OR2J2, the gene coding for OR2J2 with an optimized C-terminus (DNA sequence SEQ ID NO: 1 DNA and amino acid sequence SEQ ID NO: 2) was synthesized by a DNA synthesis service provider (BioCat GmbH, Germany) and inserted into pcDNA3.1(+) using BamHl and Notl restriction sites (Invitrogen, MA, USA) downstream of the CMV promoter sequence (SEQ ID NO: 3) and before the bgh terminator sequence (SEQ ID NO: 4). The synthetic OR2J2 nucleotide sequence further contained at its 3'-end a nucleotide sequence encoding a signal peptide (mmLucy-FLAG-rho) (SEQ ID NO: 5 and 6). This plasmid thus contains a constitutively expressed OR gene.

Expression of the OR2J2 genes was performed in HEK293T cells that had been stably transfected with a DNA sequence coding for functional variants of the human RTP1S (V227I) (SEQ ID NO: 7 and 8) and RTP2(L220R) (SEQ ID NO: 9 and 10). These cells were seeded into polyethyleneimine coated 96-well plates (100µl/well) at a density of 10,000 cells/well and grown at 37°C in presence of 5% CO2 for 24 h. Growth medium is DMEM containing 9% fetal bovine serum (FBS) and penicillin / streptomycin (GibcoTM, ThermoFisher Scientific, MA, USA)

0.625 µg of the OR2J2 expression plasmid, 1 µg of the empty pcDNA3.1(+) vector, and 1 µg of pGL4.29 (Promega) harbouring the CRE-inducible luciferase were diluted in 0.25 ml OptiMEM medium (GibcoTM, ThermoFisher Scientific, MA, USA). In parallel 15 µl Lipofectamine 2000 (Invitrogen) were diluted in 0.25 ml OptiMEM medium and after 5 min preincubation, the two mixtures were combined to prepare the transfection mixture, which was incubated for an additional 25 min.

50 µl of growth medium was replaced with fresh DMEM containing 9% fetal bovine serum (FBS) and penicillin / streptomycin. The pre-incubated transfection mixture was diluted to 5.5 ml in OptiMEM medium and 50 µl of the diluted mixture was added per well (final total volume is 150 µl). Cells were further incubated for 24 h at 37°C in presence of 5% CO2 to allow for DNA uptake and expression of OR2J2.

Activation of OR2J2 by the test compounds was measured by removal of 100 µl growth medium and addition of 50 µl DMEM containing 9% FBS, penicillin / streptomycin, 1% DMSO, and different concentrations of the test substances. After incubation for 4.5 h, the cells were lysed using 20 µl passive lysis buffer (Promega) and the luciferase signal, which is induced based on OR-dependent cAMP production, was measured.

### Sequences:

OR2J2 DNA sequence with an optimized C-terminus DNA sequence (SEQ ID NO 1): 
ATGATGATTAAAAAAAATGCAAGTTCGGAAGACTTCTTTATTCTACTTGGATTTTCTAATT
GGCCTCAGCTGGAAGTAGTTCTCTTTGTGGTTATCTTGATCTTCTACCTGATGACACTGA
CAGGAAACCTGTTCATCATCATCCTGTCATACGTGGACTCCCATCTCCACACACCAATGT
ACTTCTTCCTTTCAAACCTCTCATTTCTGGATCTCTGCTACACCACCAGCTCTATCCCTCA
GTTGCTGGTGAATCTCCGGGGCCCGGAAAAGACCATCTCGTATGCTGGTTGCATGGTTC
AACTTTACTTTGTTCTTGCACTGGGAATCGCAGAGTGTGTCCTACTGGTGGTGATGTCGT
ATGATCGTTATGTAGCTGTGTGTAGACCTTTGCATTACACTGTCCTCATGCACCCTCGTT
TCTGCCACTTGTTGGTTGCGGCTTCTTGGGTAATTGGTTTTACTATCTCAGCACTTCATTC
CTCCTTTACTTTCTGGGTACCCCTTTGTGGACATCGCCTAGTGGATCACTTCTTCTGTGA
AGTTCCAGCACTTCTGCGTTTATCATGTGTTGACACCCATGCAAATGAGCTGACCCTCAT
GGTCATGAGCTCCATTTTTGTTCTCATACCTCTCATTCTGATTCTCACTACCTATGGTGCC
ATTGCCCGGGCTGTACTGAGCATGCAATCAACCACTGGGCTTCAGAAAGTGTTTAGGAC
ATGTGGAGCCCATCTTATGGTTGTATCTCTCTTTTTCATTCCAGTCATGTGCATGTATCTC
CAGCCACCATCAGAAAATTCTCCTGATCAGGGCAAGTTCATTGCCCTCTTTTATACTGTT
GTCACACCGAGTCTTAATCCTCTAATCTACACTCTCAGGAACAAAGAAGTTAAAAAGGCC
ATAAAGAGGTTGTTCAAGAGAAAGTGCTGCAGGAGAAGGTGA
OR2J2 amino acid sequence with an optimized C-terminus amino acid sequence (SEQ ID NO 2): 
MMIKKNASSEDFFILLGFSNWPQLEVVLFVVILIFYLMTLTGNLFIIILSYVDSHLHTPMYFFLSN
LSFLDLCYTTSSIPQLLVNLRGPEKTISYAGCMVQLYFVLALGIAECVLLVVMSYDRYVAVCRP
LHYTVLMHPRFCHLLVAASWVIGFTISALHSSFTFWVPLCGHRLVDHFFCEVPALLRLSCVDT
HANELTLMVMSSIFVLIPLILILTTYGAIARAVLSMQSTTGLQKVFRTCGAHLMVVSLFFIPVMC
MYLQPPSENSPDQGKFIALFYTVVTPSLNPLIYTLRNKEVKKAIKRLFKRKCCRRR
CMV Promoter DNA sequence (SEQ ID NO 3) 
GTTGACATTGATTATTGACTAGTTATTAATAGTAATCAATTACGGGGTCATTAGTTCATAG
CCCATATATGGAGTTCCGCGTTACATAACTTACGGTAAATGGCCCGCCTGGCTGACCGC
CCAACGACCCCCGCCCATTGACGTCAATAATGACGTATGTTCCCATAGTAACGCCAATA
GGGACTTTCCATTGACGTCAATGGGTGGAGTATTTACGGTAAACTGCCCACTTGGCAGT
ACATCAAGTGTATCATATGCCAAGTACGCCCCCTATTGACGTCAATGACGGTAAATGGCC
CGCCTGGCATTATGCCCAGTACATGACCTTATGGGACTTTCCTACTTGGCAGTACATCTA
CGTATTAGTCATCGCTATTACCATGGTGATGCGGTTTTGGCAGTACATCAATGGGCGTG
GATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTCCGCCCCATTG
ACGCAAATGGGCGGTAGGCGTGTACGGTGGGAGGTCTATATAAGCAGAGCTC
bgh terminator DNA sequence (SEQ ID NO 4) 
CTGTGCCTTCTAGTTGCCAGCCATCTGTTGTTTGCCCCTCCCCCGTGCCTTCCTTGACC
CTGGAAGGTGCCACTCCCACTGTCCTTTCCTAATAAAATGAGGAAATTGCATCGCATTGT
CTGAGTAGGTGTCATTCTATTCTGGGGGGTGGGGTGGGGCAGGACAGCAAGGGGGAG
GATTGGGAAGACAATAGCAGGCATGCTGGGGATGCGGTGGGCTCTATGG
mmLucy-FLAG-rho DNA sequence (SEQ ID NO 5) 
ATGAGCCACCAGATCCTGCTGCTCCTGGCCCTGCTGACCCTAGGCCTGGCTGATTACAA
GGACGACGACGATAAGATCGAATTGATGAACGGGACCGAGGGCCCAAACTTCTACGTG
CCTTTCTCCAACAAGACGGGCGTGGTGGAATTC
mmLucy-FLAG-rho amino acid sequence (SEQ ID NO 6)
   MSHQILLLLALLTLGLAMDYKDDDDKMNGTEGPNFYVPFSNKTGVVEF
RTP1S(V227I) DNA sequence (SEQ ID NO 7) 
ATGTGTAAAAGCGTGACCACAGATGAGTGGAAGAAAGTCTTCTATGAGAAGATGGAGGA
GGCAAAGCCGGCTGACAGCTGGGACCTCATCATAGACCCCAACCTCAAGCACAATGTG
CTGAGCCCTGGTTGGAAGCAGTACCTGGAATTGCATGCTTCAGGCAGGTTCCACTGCTC
CTGGTGCTGGCACACCTGGCAGTCGCCCTACGTGGTCATCCTCTTCCACATGTTCCTGG
ACCGCGCCCAGCGGGCGGGCTCGGTGCGCATGCGCGTCTTCAAGCAGCTGTGCTATG
AGTGCGGCACGGCGCGGCTGGACGAGTCCAGCATGCTGGAGGAGAACATCGAGGGCC
TGGTGGACAACCTCATCACCAGCCTGCGCGAGCAGTGCTACGGCGAGCGTGGCGGCC
AGTACCGCATCCACGTGGCCAGCCGCCAGGACAACCGGCGGCACCGCGGAGAGTTCT
GCGAGGCCTGCCAGGAGGGCATCGTGCACTGGAAGCCCAGCGAGAAGCTGCTGGAGG
AGGAGGCGACCACCTACACCTTCTCCCGGGCGCCCAGCCCCACCAAGTCGCAGGACCA
GACGGGCTCAGGCTGGAACTTCTGCTCTATCCCCTGGTGCTTGTTTTGGGCCACGGTCC
TGCTGCTGATCATCTACCTGCAGTTCTCTTTCCGTAGCTCCATCTAA
RTP1S(V227I) amino acid sequence (SEQ ID NO 8) 
MCKSVTTDEWKKVFYEKMEEAKPADSWDLIIDPNLKHNVLSPGWKQYLELHASGRFHCSW
CWHTWQSPYVVILFHMFLDRAQRAGSVRMRVFKQLCYECGTARLDESSMLEENIEGLVDNL
ITSLREQCYGERGGQYRIHVASRQDNRRHRGEFCEACQEGIVHWKPSEKLLEEEATTYTFS
RAPSPTKSQDQTGSGWNFCSIPWCLFWATVLLLIIYLQFSFRSSI
RTP2(L220R) DNA sequence (SEQ ID NO 9) 
ATGTGTACCAGCTTGACCACTTGTGAGTGGAAGAAAGTCTTCTATGAGAAGATGGAGGT
GGCAAAGCCAGCGGACAGCTGGGAGCTCATCATAGACCCCAACCTCAAGCCCAGTGAG
CTGGCCCCTGGCTGGAAGCAGTACCTGGAGCAGCACGCCTCAGGCAGGTTCCACTGCT
CCTGGTGCTGGCACACCTGGCAGTCTGCCCATGTGGTCATCCTCTTCCACATGTTCCTG
GACCGCGCCCAGCGGGCGGGCTCGGTGCGCATGCGCGTCTTCAAGCAGCTGTGCTAT
GAGTGCGGCACGGCGCGGCTGGACGAGTCCAGCATGCTGGAGGAGAACATCGAGGGC
CTGGTGGACAACCTCATCACCAGCCTGCGCGAGCAGTGCTACGAGGAGGATGGTGGCC
AGTACCGCATCCACGTGGCCAGCCGCCCGGACAGCGGGCCGCATCGTGCAGAGTTCT
GTGAGGCCTGCCAGGAGGGCATCGTTCACTGGAAGCCCAGCGAGAAGCTGCTGGAGG
AGGAGGTGACCACCTACACCTCTGAAGCCTCCAAGCCGAGGGCCCAGGCGGGATCCG
 
GCTACAACTTCTTGTCTCTTCGCTGGTGCCTCTTCTGGGCCTCTCTCTGCCTGCTCGTTG
TTTACCTGCAGTTCTCCTTCCGCAGTCCTGCCTTCTTTTAG
RTP2(L220R) amino acid sequence (SEQ ID NO 10)

MCTSLTTCEWKKVFYEKMEVAKPADSWELIIDPNLKPSELAPGWKQYLEQHASGRFHCSW
CWHTWQSAHVVILFHMFLDRAQRAGSVRMRVFKQLCYECGTARLDESSMLEENIEGLVDNL
ITSLREQCYEEDGGQYRIHVASRPDSGPHRAEFCEACQEGIVHWKPSEKLLEEEVTTYTSEA
SKPRAQAGSGYNFLSLRWCLFWASLCLLVVYLQFSFRSPAFF

## Claims

1. A compound of formula (I) 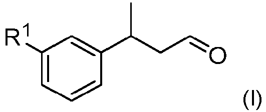 wherein R¹ is selected from n-propyl, n-butyl, n-pentyl, n-hexyl and n-heptyl.

2. The compound according to claim 1 selected from the group consisting of 3-(3-propylphenyl)butanal, 3-(3-butylphenyl)butanal, 3-(3-pentylphenyl)butanal, 3-(3-hexylphenyl)butanal and 3-(3-heptylphenyl)butanal.

3. The compound according to claim 1 selected from the group consisting of 3-(3-pentylphenyl)butanal and 3-(3-hexylphenyl)butanal.

4. Use of the compound of formula (I) according to claim 1as fragrance compound 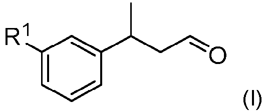 wherein R¹ is selected from n-propyl, n-butyl, n-pentyl, n-hexyl and n-heptyl, as fragrance.

5. A fragrance composition comprising
i) at least one compound of formula (I) 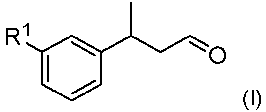 wherein R¹ is selected from n-propyl, n-butyl, n-pentyl, n-hexyl and n-heptyl;
ii) and at least one other odorant.

6. A consumer product comprising
i) at least one compound of formula (I) 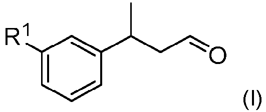 wherein R¹ is selected from n-propyl, n-butyl, n-pentyl, n-hexyl and n-heptyl;
ii) and a consumer product base.

7. A method for improving, enhancing or modifying a consumer product base by means of the addition thereto a compound of formula (I), or a mixture thereof 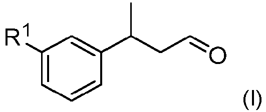 wherein R¹ is selected from n-propyl, n-butyl, n-pentyl, n-hexyl and n-heptyl.
